# EUROPEAN PATENT APPLICATION

(11) **EP 4 438 194 A1**
(43) Date of publication of application: **02.10.2024**
(21) Application number: 22896856.6
(22) Date of filing: 16.11.2022
(51) Int. Cl.: B09B 3/65

(54) **DEVICE FOR THE CLEANING AND MORPHOLOGICAL TRANSFORMATION OF WASTE TO MAKE SAME MANAGEABLE**

(30) Priority: 25.11.2021 ES 202131096
(71) Applicant: Thermowaste, S.L., 08401 Granollers (Barcelona) (ES)
(72) Inventor: RIBAS MARTINEZ, Oscar, 08401 Granollers ( BARCELONA) (ES); RIBAS LOPEZ, Rafael, 08401 Granollers ( BARCELONA) (ES)
(74) Representative: Espiell Gomez, Ignacio
(86) International application number: PCT/ES2022/070737
(87) International publication number: WO 2023/094719

(57) **Abstract**

The present invention relates to an equipment for cleaning and morphological transformation of wastes so that same are manageable, comprising at least one reactor (1) made up of several modules (2a, 2b, 2c, 2d) that can be disassembled and coupled together, each of which internally incorporates means to carry out a specific function that, at least, covers the function of loading, cleaning and morphological transformation of process wastes, discharge and dehydration, and control and electrical power. Preferably, the modules are four modules consisting of: a loading module (2a), a module that contains the process reactor body (2b), a discharge (2c) and dehydration module, and a control (2d) and power module and same are removable, are manufactured with measurements and exterior design meeting the ISO 20 and ISO 40 requirements stipulated for maritime containers or the like.

## Description

### OBJECT OF THE INVENTION

The invention, as expressed in the statement of this specification, relates to an equipment for cleaning and morphological transformation of wastes so that same are manageable which provides, to the function for which it is intended, advantages and features, which are described in detail below.

For the purposes of this document, the process of cleaning and morphological transformation of wastes so that same are manageable, the object of the invention, will be identified as the cleaning-for-managing process.

The process of cleaning and morphological transformation of wastes so that same are manageable (cleaning-for-managing process) is a technique through which municipal solid waste, industrial waste comparable to urban waste and hospital waste or other waste, are transformed into clean and manageable materials, for its subsequent use in the form of by-products or raw materials, avoiding the dumping thereof in landfills, uncontrolled dumps in fields, rivers or seas, its destruction in incinerators, avoiding the harmful impact on the environment and promoting the circular economy. The process of cleaning and morphological transformation of wastes so that same are manageable (cleaning-for-managing process), object of the invention, mainly combines the simultaneous actions of sterilisation, morphological transformation, homogenisation of biodegradable fractions, tearing of cellulose fibres, and moisture transfer between fractions of materials, depigmentation of metals and plastic shrink-wrapping.

The object of the present invention lies in a process for cleaning and morphological transformation of wastes so that same are manageable (cleaning-for-managing process), which is essentially distinguished by comprising a base processing unit, which will be called reactor herein, made up of several removable modules, preferably with dimensions and exterior design meeting the requirements stipulated for maritime containers, each of which incorporates the means to perform a specific function; such as loading, cleaning and morphological transformation of wastes, unloading and dehydration, support structure and logistics, as well as control and electrical power.

### FIELD OF APPLICATION OF THE INVENTION

The field of application of the present invention falls within the industry sector dedicated to the processing and transformation of wastes, mainly urban solid waste, waste comparable to urban waste and hospital waste.

### BACKGROUND OF THE INVENTION

As a reference to the current state of the art, it should be noted that, although different facility types and models intended for the processing and transformation of urban solid waste are known, at least the applicant is not aware of the existence of any designed as a transportable modular equipment, nor any that presents technical, structural and constitutive features that are the same or similar to those presented by the equipment claimed herein.

### DESCRIPTION OF THE INVENTION

The equipment for cleaning and morphological transformation of waste so that same are manageable (cleaning-for-managing process) that the invention proposes is configured as an advantageous alternative to the facilities currently known for the same purpose, the characterising details that make it possible and that distinguish it being conveniently included in the final claims that accompany this description.

Specifically, what the invention proposes, as noted above, is an equipment that can be used to carry out the processing and transformation of municipal solid wastes, to convert same into clean and manageable materials, which can be introduced back into the market, thus avoiding the disposal thereof in landfills or its destruction in incinerators and consequently the negative impact on the environment, which is essentially distinguished by the fact that it is a modular and easy to transport assembly, for which it basically comprises a base processing unit or reactor comprising several removable modules that can be coupled together, each of them being equipped with the means required to carry out a specific function of said processing and transformation, specifically, the function of loading the waste, that of treating or cleaning and managing of the waste, the unloading and dehydration of the waste, and the support and logistics structure, as well as control and electrical power.

The system is specially designed for the processing of municipal solid waste, certain industrial waste similar to urban waste, organic waste from industrial processes, wastes from clinics and hospitals, wastes contaminated by SARS-CoV and other pathogens, among others.

Furthermore, in the preferred embodiment, said modules are manufactured with measurements and exterior design meeting the requirements stipulated for ISO 20 and ISO 40 maritime containers, in order to facilitate the transportation thereof by sea and land means in accordance with accepted standards on the market and its many variants.

Preferably, said reactor comprises removable modules the assembly of which constitutes an autonomous machine with the capacity to process between 10 and 300 tons of waste per day.

Each module, although externally it has the appearance of a maritime container, develops a specific function and is an intrinsic portion of what the reactor as a whole represents, playing a relevant role in its operation. Thus, the parts of the reactor are: a first loading module, a second module that contains the reactor body that executes the process of cleaning and morphological transformation of wastes, a third discharge and dehydration module, and a fourth support structure and logistics module, which also preferably includes the control and power supply area.

The wastes are preferably collected by means of a hopper and transferred, preferably, by means of a conveyor belt to a feed opening provided for this purpose in the loading module of the reactor. There, the waste is deposited and channelled for processing thereof. The loading module is formed by a sealed compression unit that crushes and compresses the waste, while introducing same into the module mounted downstream that contains the reactor body that executes the process of cleaning and morphological transformation of wastes.

In the module that contains the reactor body, the simultaneous process of cleaning and morphological transformation of wastes is carried out by applying several actions simultaneously so that the waste is turned into clean and manageable waste.

By way of example and not as a limitation, in said module that contains the reactor body, the waste is torn apart, the plastic bags are opened, they are subjected to a high-pressure steam bath, they are stabilised, sanitised, and cellulose fibres are torn, plastics are shrink-wrapped, moisture transfers occur between fractions of the different materials and their morphology is homogenised. The sum of all these operations, which are carried out using apparatuses, devices and tools designed for this purpose, makes up the cleaning-for-managing process and may involve remaining inside for a period of time between 10 and 60 minutes.

At the end of the processing time, the resulting materials are introduced into the discharge and dehydration module. There they are prepared for extraction from the reactor in the form of clean and manageable materials.

A mechanical conveying system dispenses the same through an outlet opening to feed another external belt conveyor. This last belt conveyor, which is no longer part of the equipment in question, allows the transport of already processed materials to the classification area, where a set of automatic operations, such as a sieve, an optical separator or an electromagnet, or by manual means, allow each and every fraction of materials to be obtained that will later be exploited in the form of by-products.

It is a continuous process, that is, the waste is introduced into the reactor through the first loading module and is extracted constantly and continuously over time without interruptions 24 hours a day, beyond scheduled shut-downs or untimely stoppages that may occur as a result of breakdowns or obstructions.

The additional functions and advantages of the structures that define the described modules that make up the reactor comprising the equipment object of the invention are the following:
- They allow the equipment to be transported in parts (modules) complying with ISO 20 and ISO 40 or similar standards.
- They facilitate the assembly process at destination, since each module incorporates connection hooks for connecting to adjacent modules.
- They facilitate the disassembly and transfer of the reactor to another location.
- The shell (skin) of each module, made of coated metal sheet, prevents access to the interior of the equipment, to prevent the theft of components during the transport thereof.
- The shell (skin) of each module prevents access to parts of the equipment that could put people's health and safety at risk. Each module is equipped with a series of access gates or manholes to areas that can be used for technical maintenance interventions. These gates incorporate security means for opening and sensors for remote control and supervision.
- The shell (skin) of each module allows the internal temperature of the module and heat dissipation to be regulated, thus reducing energy consumption, since they are preferably made of heat-insulating materials.
- The shell (skin) of each module allows, on its exterior face, to incorporate badges and informative and/or advertising messages from the client, which have a visual and positive impact for commercial promotion purposes.

Preferably, the advancement of the material in the module containing the reactor body is carried out by means of an inclination of between 0.1 and 5 degrees.

Preferably, not only the module containing the reactor body is tilted, but rather the entire reactor is tilted such that it acts as a single unit when assembled, the entire reactor (the set of preferably four modules) being that which is simultaneously tilted with respect to the ground.

To do this, preferably, before proceeding to assemble the reactor in its final location, metal bases are installed to ensure a flat settlement despite the irregularities of the terrain. The discharge and control modules are supported on these metal bases, which preferably go below the loading modules and the module that contains the reactor body. And, subsequently, said metal bases are subjected to an adjustment process to achieve the desired angle of inclination. After this, the loading modules and the module containing the reactor body are incorporated from above.

Another important function of the team's modular reactor structures is to control the directions of expansion of the module containing the reactor body. The interior components of the module that contains the reactor body can expand several centimetres longitudinally once normal process conditions are reached. The structure of this module that contains the reactor body regulates the directions of expansion of the components same contains. Likewise, the specific design of the loading module structure makes it possible to adsorb the expansion that occurs in the module that contains the reactor body.

Once the equipment is installed, the control module acts as a cockpit. It contains the electrical power panels, the equipment control panels and other elements intended to operate the valves and pneumatic actuators.

It should be noted that, during transport of the reactor, certain components that protrude beyond the clearance of the four modules must be dismantled in order to comply with current transport regulations on maritime containers. All these elements are transported inside the control module. To do this, said control module incorporates a series of specific hooks to retain these removable components and prevent the displacement thereof during the trip.

One of the priorities in the design of the equipment object of the invention is to guarantee maximum availability. When a city or region stops sending its waste to a landfill to send it to a facility based on the modular equipment object of this invention, same must guarantee its availability for the greatest number of hours possible throughout the year, given that the garbage does not stop being generated.

To do this, any breakdown that may arise unexpectedly must be resolved as quickly as possible and, in the same manner, preventive or corrective maintenance actions must be able to be carried out in the shortest possible time. To this end, different levels of intervention have been devised, which involve the replacement of affected parts or components, or in the most extreme cases, the complete replacement of a damaged module with another identical one in operating conditions. When it comes to replacing parts or components, the structure of the modules plays a fundamental role, incorporating support and anchoring points to lift and move by means of hoists those elements that an operator cannot support or lift with their own hands. That is, the structure of each module is also an essential tool for maintenance activities.

### DESCRIPTION OF THE DRAWINGS

To complete the description, and for the purpose of helping to make the features of the invention more readily understandable, this description is accompanied by a set of drawings constituting an integral part of the same, wherein by way of illustration and not limitation the following has been represented:
Figure number 1 shows a schematic representation in perspective of the equipment for cleaning and morphological transformation of wastes so that same are manageable (cleaning-for-managing process) object of the invention, where the different modules that make it up can be seen; and
figures number 2, 3 and 4 show different perspectives, taken from different points of view, of an exemplary embodiment of the complete assembly of the equipment for cleaning and morphological transformation of wastes so that same are manageable (cleaning-for-managing process), according to the invention, showing the parts and elements that it comprises, as well as their configuration and arrangement along with certain auxiliary elements, such as loading and unloading belts or a sieve.

### PREFERRED EMBODIMENT OF THE INVENTION

In view of the aforementioned figures, and in accordance with the adopted numbering, one may observe therein a non-limiting exemplary embodiment of the equipment for cleaning and morphological transformation of waste so that same are manageable (cleaning-for-managing process) that is the object of the invention, comprising that which is described in detail below.

Thus, as seen in said figures, the equipment of the invention comprises at least one reactor (1) made up of several removable modules (2a, 2b, 2c, 2d) that are coupled together, each of which internally incorporates means to carry out a specific function that, at least, covers the function of loading, cleaning and morphological transformation of wastes, discharge and dehydration, and control and electrical power.

Preferably, said removable modules 2a, 2b, 2c, 2d) are manufactured with measurements and exterior design meeting the requirements ISO 20 and ISO 40 stipulated for maritime containers or the like.

Preferably, the reactor (1) comprises four modules consisting of: a loading module (2a), a module that contains the process reactor body (2b), a discharge (2c) and dehydration module, and a control (2d) and power module.

Optionally, the equipment object of the invention comprises a series of auxiliary elements, such as a hopper (3) where the wastes are collected and a rolling belt (4) in which it is transported to a feeding mouth (5) provided for this purpose in the loading module (2a) of the reactor (1).

Preferably, the loading module (2a) is formed by an equipment that compresses and introduces the waste into the subsequent module (2b) to be processed.

Preferably, the module that contains the body of the reactor (2b) for cleaning and handling comprises means consisting of different apparatus, devices and/or tools for processing and treating wastes such that, by way of example and not limitation, they include means that tear waste apart, means that open plastic bags, means for subjecting waste to a high-pressure steam bath, means for stabilising and sanitising waste, means that disintegrate cellulose fibres, means that transfer moisture between fractions of materials, means that depigment metals, means that shrink-wrap plastics and means that homogenise the morphology thereof.

Preferably, the discharge and dehydration module (2c) from which the waste is extracted from the reactor (1) in the form of clean and manageable materials, comprises an outlet opening (6) that feeds an auxiliary belt (7) which, in turn, allows the materials to be transported to the classification area, where a set of automatic processes, for example a sieve (8), or manual ones, allow each and every one of the fractions of material to be obtained that are subsequently exploited in the form of by-products.

Preferably, each module (2a, 2b, 2c, 2d) that makes up the reactor (1) incorporates connection hooks for connecting to the adjacent modules.

Preferably, the shell of the modules (2a, 2b, 2c, 2d) is made of metal sheet with thermal insulation coating, and is equipped with access gates (9) for areas that can be used for technical maintenance interventions, which incorporate appropriate security means for opening and sensors for remote control and supervision.

Preferably, the modules (2a, 2b, 2c, 2d) of the reactor (1) are mounted with a certain inclination, between 0.1 and 5 degrees, with respect to the horizontal plane so that the advance of the material is carried out due to the effect of gravity thanks to said inclination.

To do this, preferably, before proceeding with the assembly of the reactor (1) at the placement thereof, metal bases must be installed to ensure a flat settlement despite the irregularities of the terrain, on which the discharge (2c) and control (2d) modules rest, preferably installed below the loading module (2a) and the module that contains the reactor body (2b).

The structure of the module that contains the reactor body (2b) is such that it regulates the directions of expansion of the interior components of said module that contains the reactor body (2b) and that they can expand several centimetres longitudinally once the normal process conditions are reached. Likewise, the structure of the loading module (2a) is such that it allows the expansion that occurs in the module that contains the reactor body (2b) to be adsorbed.

Preferably, the control module (2d) acts as a cockpit and contains the electrical power panels, the equipment control panels and other elements intended to operate the valves and pneumatic actuators.

Furthermore, preferably, the control module (2d) defines a support and logistics structure, by having space for the transport of some removable components of the equipment, as well as specific hooks to retain these removable components inside and prevent the displacement thereof during transport.

Having sufficiently described the nature of the present invention, as well as the ways in which it may be implemented, it is not considered necessary to elaborate on the explanation thereof in order for a person skilled in the art to understand the scope of the invention and the advantages derived therefrom.

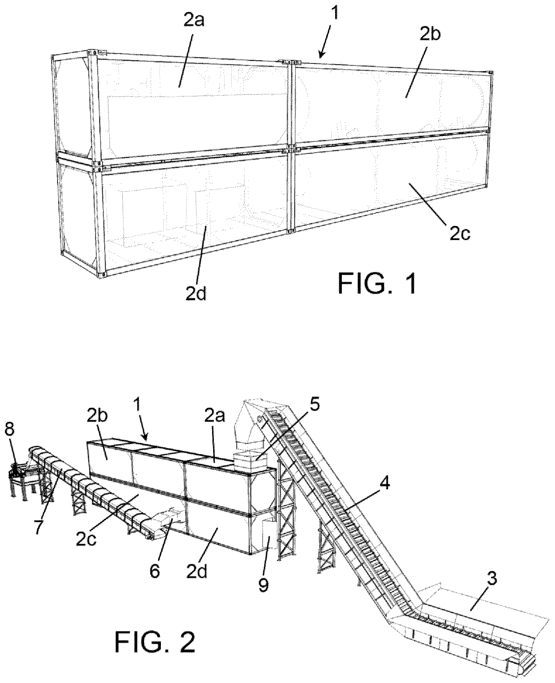

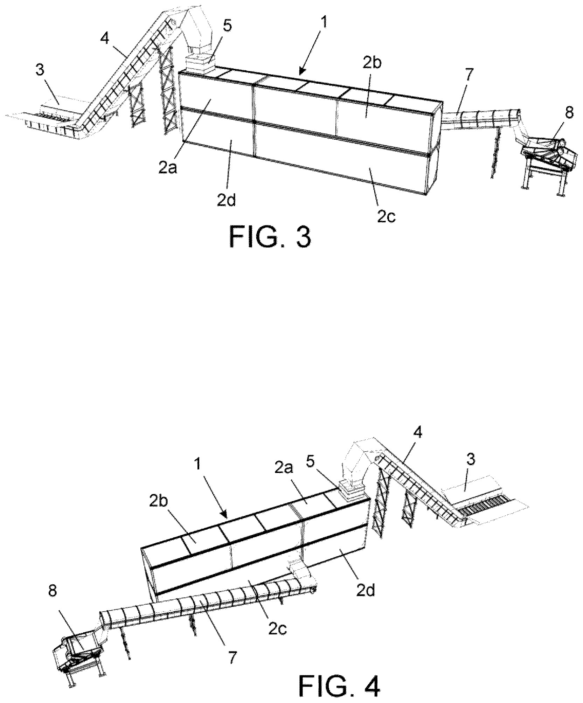

## Claims

1. An equipment for cleaning and morphological transformation of wastes so that same are manageable, such as municipal solid waste, industrial waste comparable to urban waste, and hospital waste, **characterised in that** it comprises, at least, one reactor (1) which in turn comprises
• a loading module (2a), which constitutes a sealed compression unit that compresses the waste and introduces same into the adjacent module,
• a module that contains the reactor body (2b) that executes the process of cleaning and morphological transformation of wastes,
• a discharge (2c) and dehydration module of the already clean and manageable waste, and
• a control module (2d) and power supply
said modules (2a, 2b, 2c, 2d) being removable, attachable to each other, with measurements and exterior design meeting the requirements stipulated for maritime containers and said modules (2a, 2b, 2c, 2d) being covered by a shell equipped with a series of access gates or manholes to areas that can be used for technical maintenance interventions.

2. The equipment for cleaning and morphological transformation of wastes so that same are manageable, according to claim 1, **characterised in that** the removable modules (2a, 2b, 2c, 2d) are manufactured with measurements and exterior design meeting the ISO 20 and ISO 40 requirements stipulated for maritime containers or the like.

3. The equipment for cleaning and morphological transformation of wastes so that same are manageable, according to claim 1, **characterised in that** the module that contains the reactor body (2b) comprises means that, consisting of different apparatuses, devices and/or tools for processing and treating wastes, comprises means that tear waste apart, means that open plastic bags, means for subjecting waste to a high-pressure steam bath, means for stabilising and sanitising waste, means that disintegrate cellulose fibres, means that transfer moisture between fractions of material, means that depigment metals, means that shrink-wrap plastics and means that homogenise the morphology thereof.

4. The equipment for cleaning and morphological transformation of wastes so that same are manageable, according to claim 1, **characterised in that** the discharge (2c) and dehydration module from which the waste is expelled out of the reactor (1) in the form of clean materials includes an outlet opening (6) that feeds an external belt (7) which, in turn, allows the materials to be transported to the classification area.

5. The equipment for cleaning and morphological transformation of wastes so that same are manageable, according to any of the preceding claims, **characterised in that** each module (2a, 2b, 2c, 2d) that makes up the reactor (1) incorporates connection hooks for connecting to the adjacent modules.

6. The equipment for cleaning and morphological transformation of wastes so that same are manageable, according to any of the preceding claims, **characterised in that** the shell of the modules (2a, 2b, 2c, 2d) is made of coated metal sheet, preferably thermal insulation, and is equipped with access gates (9) for areas that can be used for technical maintenance interventions, which incorporate security means for opening and sensors for remote control and supervision.

7. The equipment for cleaning and morphological transformation of wastes so that same are manageable, according to any of the preceding claims, **characterised in that** the modules (2a, 2b, 2c, 2d) of the reactor (1) are mounted with an inclination of between 0.1 and 5 degrees, with respect to the horizontal plane such that the advance of the material is carried out due to the effect of gravity thanks to said inclination.

8. The equipment for cleaning and morphological transformation of wastes so that same are manageable, according to any of claims 2 to 7, **characterised in that** the structure of the module that contains the reactor body (2b) regulates the directions of expansion of the interior components of said module that contains the reactor body (2b) and which can expand several centimetres longitudinally once the normal process conditions are reached.

9. The equipment for cleaning and morphological transformation of wastes so that same are manageable, according to any of claims 2 to 8, **characterised in that** the control module (2d) acts as a cockpit and it contains the electrical power panels, the equipment control panels and other elements intended to operate the valves and pneumatic actuators.

10. The equipment for cleaning and morphological transformation of wastes so that same are manageable, according to any of claims 2 to 9, **characterised in that** the control module (2d) defines a support and logistics structure with space for the transport of some removable components of the equipment as well as specific hooks to retain these removable components and prevent the displacement thereof during the trip or transfer.
